# EUROPEAN PATENT SPECIFICATION

(11) **EP 2 583 634 B1**
(45) Date of publication and mention of the grant of the patent: **09.03.2016**
(21) Application number: 11008466.2
(22) Date of filing: 21.10.2011
(51) Int. Cl.: A61B 90/00, A61B 90/18, A61B 6/04

(54) **Head immobilization device**
Kopfimmobilisierungsvorrichtung
Dispositif d'immobilisation de tête

(43) Date of publication of application: 24.04.2013
(73) Proprietor: MIS Medical Imaging Systems GmbH, 82402 Seeshaupt (DE)
(72) Inventor: Al-kassim, Omar, 2200 Copenhagen N (DK); Al-kassim, Adil, 2200 Copenhagen N (DK)
(74) Representative: Grünecker Patent- und Rechtsanwälte PartG mbB

(56) References cited:
- DE-U1- 29 813 194
- DE-U1-202011 001 492
- US-A- 5 775 337
- US-A1- 2002 038 659

## Description

### Field of the Invention

The present invention relates to a head immobilization device used in the medical field having the features of the preamble part of claim 1.

### Background of the Invention

As a state of the art, head immobilization devices are known in the medical field to immobilize a patient's head during medical treatment, since it is necessary that the patient's head remains in the same position during the whole course of the medical treatment. The head immobilization device comprises a support plate structure and a head fixation element, whereby the head fixation element can be releasably connected to the support plate structure. During the medical treatment, the support plate structure serves as a head rest for the posterior head portion, while the anterior head portion is covered tightly with the head fixation element.

The head fixation element comprises an open frame element with a mask element attached to it. The open frame element serves as a stabilization element for the mask element as well as an interface to the support plate structure. The mask element is fitted individually to the anterior head portion of a patient, e.g. by a molding process. As an example, the mask element can be molded directly to the anterior head portion by means of a net-like low temperature thermoplastic, with a working temperature between 65 - 72°C.

The support plate structure incorporates a fixture mechanism comprising a base fixture element and leg fixture elements for fixing the base portion and the legs of the open frame element respectively. A levering mechanism with a spring load is provided in the support plate structure to actuate the base fixture element. When the head fixation element is attached or released, the operator levers the base fixture element with one hand into an opened state by applying a manual force on the levering mechanism against the spring load. The head fixation element can then be removed or attached with the other hand of the operator.

It has been shown, that the levering mechanism is complex during attachment and release operation. Thereby discomfort for the patient is caused.
Furthermore, Document US 5,755,337 A discloses an immobilization device; and
Document US 2002/0038659 A1 discloses an immobilization system.

### Summary of the Invention

It is the object of the invention to provide a more simplified fixing mechanism, which can be handled more easily.

The present invention provides a head immobilization device for use in the medical field, comprising:
The object is met with a device, having the features of the preamble part of claim 1, and being characterized in that
the at least two leg fixture elements are designed to cooperate with the end portions of the legs such as to bias the head fixation element in the direction of the at least one base fixture element.

The attachment operation of the present invention is easier, since therefore the end portions of the open frame element can be inserted into the at least two leg fixture elements and can be forced against spring-like elements within the at least two fixture elements. With a single hand or both hands on the open frame element, the open frame element can be latched into the at least one base fixture element. The release operation is easier, since therefore the open frame element can be unlatched from the at least one base fixture element by forcing the open frame element into the direction of the at least two leg fixture elements with a single or both hands and by applying a lifting force to remove the head fixation element. Both attachment and release operations can be carried out with a single hand or with both hands on the open frame element, thus providing more comfort for the patient.

Since the at least one base fixture element can be designed as a single part, which is fixed rigidly to the support plate structure, the fixing mechanism is simplified.

A head immobilized by the head immobilization device may comprise head portions, neck portions and/or shoulder portions of a human or an animal. The head immobilization device may be used for medical treatment. Medical treatment may comprise medical therapy and medical diagnostics. The head immobilization device may be used for radiological treatment, particularly in conjunction with tumors and/or cancerous tissue.

The support plate structure may comprise a generic head rest and/or an individual head rest adapted to an individual head. Particularly, the individual head rest may be made from a thermoplastic material, and more particularly from a low temperature thermoplastic material with a melting point in the range from 0°C to 100°C, in particular, in the range from 70°C to 100° C. The low temperature thermoplastic material may have a working temperature in the range from 0°C to 100°C, in particular, in the range from 65°C to 72° C. The support plate structure may be attached to a base structure, particularly with a hinge. The support plate structure and/or the base structure may comprise adjustment mechanisms to adjust the position and/or inclination of the head.

The open frame element may be U-shaped. U-shape may mean that the base portion and/or the legs comprise at least one curved and/or at least one linear section. The open frame element may comprise at least one hinge, particularly to expand the angle between the legs, particularly to stretch the mask element.

The mask element may comprise a non-conductive material, in particular a synthetic material, in particular a low temperature thermoplastic material. The low temperature thermoplastic material may have a melting point in the range from 0°C to 100°C, in particular, in the range from 70°C to 100° C. The low temperature thermoplastic material may have a working temperature in the range from 0°C to 100°C, in particular, in the range from 65°C to 72° C. The mask element may comprise at least one mesh portion and/or at least one hole.

The at least two leg fixture elements may comprise a guidance element and a receptacle element, said guidance element guiding the receptacle element, particularly whereby the receptacle element is movable. Particularly, the receptacle element may be designed to cooperate with the end portion of the leg.

The open frame element, the support plate structure, the base structure, the at least one base fixture element and/or the at least two leg fixture elements may comprise a non-conductive material, in particular a synthetic material, for instance plastic and/or carbon fiber.

Particularly the at least one leg fixture element may comprise an elastic element. The elastic element may be a spring, a cellular and/or foamed material. The elastic element may exert a force onto the receptacle element.

The support plate structure may comprise a release element designed to exert a counterforce on the head fixation element against the bias. Thereby the release procedure is significantly alleviated. The release element may comprise a handle element, particularly designed to be manually operated. A force exerted on the handle element by the operator may be transmitted to the head fixation element, particularly the transmitted force may be the counterforce.

Particularly, the release element may interact with the base portion of the open frame element. The release element may comprise a non-conductive material, in particular a synthetic material, for instance plastic and/or carbon fiber.

Particularly the release element may comprise a sloped surface section, which generates a lifting force on the head fixation element, when the release element is activated. Particularly the slope may generate a lifting force on the base portion of the open frame element. The lifting force may be generated by deflecting fractions of the counterforce and/or bias with the sloped surface section.

Particularly the sloped surface section may comprise the shape of at least one sector of a truncated cone. Sector may mean a sector with an angle of 360°, particularly from 10° to 180°, particularly from 60° to 120°.

The release element may comprise a pivot axis, whereby the pivot axis may comprise an offset to the axis of the truncated cone. As an example the pivot axis may be eccentric. The axis of the truncated cone may mean the axis of the at least one sector of a truncated cone. When the release element is pivoted about the pivot axis, the at least one sector of a truncated cone may swivel and/or interact with the head fixation element, in particular with the base portion of the open frame element.

The at least one base fixture element may comprise at least one profiled edge, in particularly wherein the at least one profiled edge may comprise an L-shaped and/or C-shaped profile. Profiled edge may mean, that the profiled edge is a limit stop to the base portion in at least one direction, particularly whereby the direction is perpendicular to the support plate structure. Profiled edge may mean, that a profile curve is extruded along a trajectory to form the surface of the profiled edge. The trajectory may comprise at least one straight line, circle segment and/or curve. L-shaped may mean a profile curve with two connected straight lines including an angle, particularly whereby the angle may be in the range from 0° to 180°, particularly in the range from 85° to 95°. C-shaped may mean a planar profile curve with three connected straight lines, including two angles, particularly whereby the angles may be in the range from 0° to 180, particularly in the range from 85° to 95°. Thereby the head fixation element is prevented from lifting off the base plate structure.

The at least one base fixture element may comprise at least one chamfered edge portion. Particularly the at least one base fixture element may comprise at least one inclined surface portion. The chamfered edge portion may generate an auxiliary force against the bias when the base portion is forced against the chamfered edge portion. Thereby the attachment procedure is made easier.

The base portion of the open frame element may comprise at least one contoured shoulder portion and wherein the at least one base fixture element may comprise at least one complementary contoured rim portion. At least one contoured shoulder portion may mean, that the shoulder portion is curved along the longitudinal direction of the open base portion, particularly that the at least one contoured shoulder portion exhibits a lateral kink. Particularly the at least one complementary contoured rim portion is designed to collaborate with the at least one contoured shoulder portion, particularly by a form fit. Thereby the base fixture element may provide better mounting stability to the head fixation element.

The open frame element may comprise at least one guidance notch and wherein the support plate structure and/or the base fixture element may comprise at least one positioning element, particularly wherein the at least one guidance notch may interact with the at least one positioning element. As an example the guidance notch may be V-shaped, particularly the guidance notch may comprise two edges including an angle, particularly whereby the angle may be in the range from 0° to 180°, particularly in the range from 2° to 30°. The positioning element may have complementary surfaces to the guidance notch, particularly to incorporate a form and/or force fit. Thereby the head immobilization device is positioned accurately on the support plate structure. A head fixation element for use in the previously described head immobilization device is disclosed, wherein the open frame element comprises at least one guidance notch, which interacts with the at least one positioning element.

The above and other aspects, features and advantages of the present invention will become more apparent from the following detailed description when taken in conjunction with the accompanying drawings in which:

### Brief Description of the Drawings

- Figure 1: An embodiment of an exemplary head immobilization device according to the invention;
- Figure 2: A first mounting step of the exemplary head immobilization device;
- Figure 3: A second mounting step of the exemplary head immobilization device;
- Figure 4: A third mounting step of the exemplary head immobilization device;
- Figure 5: A release step of the exemplary head immobilization device;
- Figure 6: An embodiment of a leg fixture element;
- Figure 7: An embodiment of the base fixture element;
- Figure 8: An embodiment of the release element and
- Figure 9: An embodiment of the guidance notch and the positioning element.

### Detailed Description

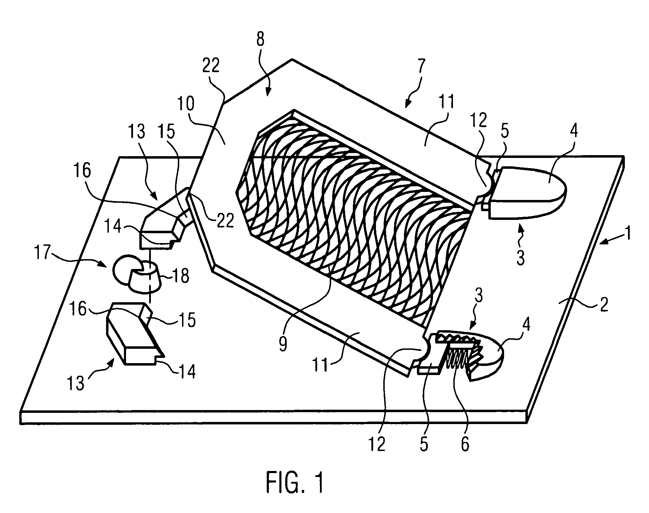

In Figure 1 parts of an embodiment of an exemplary head immobilization device are illustrated. In particular a head immobilization device is shown, which is appropriate to immobilize a patient's head during medical treatment. All parts of the head immobilization device shown in Figure 1 are manufactured from non-conductive material, e.g. carbon fiber material or plastic material, which does not interfere with the radiation and/or electromagnetic fields utilized for the medical treatment.

A support plate structure 1 is shown in Figure 1, comprising a plate element 2, leg fixture elements 3, base fixture elements 13 and a release element 17. A head fixation element 7 is shown, comprising an open frame element 8 and a mask element 9.

The open frame element 8 comprises a base portion 10 and two legs 11, each comprising two end portions 12. The base portion 10 comprises two contoured shoulder portions 22. The open frame element 8 is made from plastic material and it is connected through an adhesive with the mask element 9. The open frame element 8 stabilizes the mask element 9 and can be connected releasably to the support plate structure 1.

The mask element 9 is made from a low temperature thermoplastic material with a melting point of e.g. about 80°C. The mask element 9 has a net-like shape and is molded at a working temperature of e.g. about 65 - 72°C over the patient's anterior head, while the patient's head is in position on a not shown, known head rest of the support plate structure 1. After cooling to a temperature of e.g. about 25°C the mask element 9 stays dimensionally stable.

The plate element 2 comprises the head rest (not shown) for the posterior head portion. The leg fixture elements 3 comprise elastic elements 6, receptacle elements 5 and guidance elements 4, whereby said guidance elements 4 are fixed rigidly to the plate element 2. The receptacle element 5 is movable inside the guidance element 4 in a direction towards and away from the base fixture elements 13, whereby the receptacle element 5 can slide along four plane surfaces within the guidance element 4. The receptacle element 5 comprises a concave interfacing surface, which collaborates with the convex end portions 12 of the legs 11. The guidance element 4 and the receptacle element 5 is made for instance from plastic material and the elastic element 6 shown is a plastic spring.

The base fixture elements 13 comprise L-shaped profiled edges 14, chamfered edges 15 and complementary contoured rim portions 16. The chamfered edges 15 exert an auxiliary force on the open frame element 8 into the direction of the leg fixture elements 3, when the open frame element 8 is pressed in a downward direction against the chamfered edges 15. The base portion 10 of the open frame element 8 is fixed in position with the L-shaped profiled edges 14. The L-shaped profiled edges prevent the base portion 10 from lifting off the support plate structure 1 and from moving parallel to the plate element 2 in a direction away from the leg fixture elements 3. The complementary contoured rim portions 16 comprise as an example lateral kinks, which stabilize the open frame element 8 such that the legs 11 do not bend.

The release element 17 comprises a sloped surface section 18 to exert a lifting force on the base portion 10 of the open frame element 8, when the release element 17 is activated.

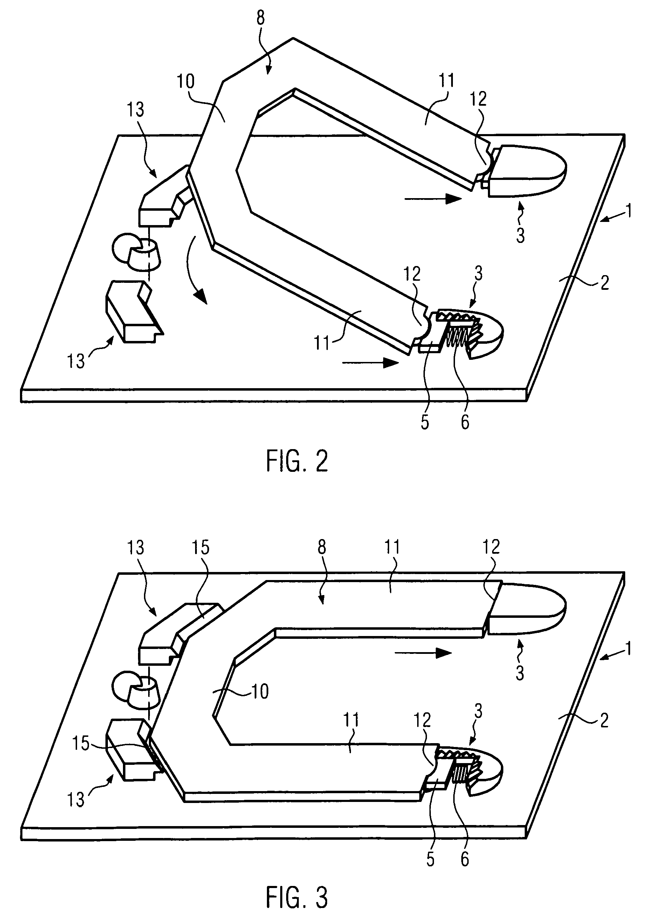

In Figure 2 the first mounting step of the exemplary head immobilization device is shown. The mask element 9 is - for ease of simplicity - not shown in the following figures.

During the first mounting step, the end portions 12 of the legs 11 of the open frame element 8 are inserted into the receptacle elements 5 of the leg fixture elements 3. The open frame element 8 is manually forced into the direction of the leg fixture elements 3, while the frame is moved downward into the direction of the support plate structure 1.

In Figure 3 the second mounting step of the exemplary head immobilization device is shown.

During the second mounting step, the open frame element 8 is pressed against the chamfered edges 15 of the base fixture elements 13 and thereby the base portions 12 are pressed versus the force of the elastic elements 6, so that the elastic elements 6 are compressed and the open frame element 8 moves towards the leg fixture elements 3. The open frame element 8 can be pushed further downward to the plate element 2, such that it is in full contact with the plate element 2.

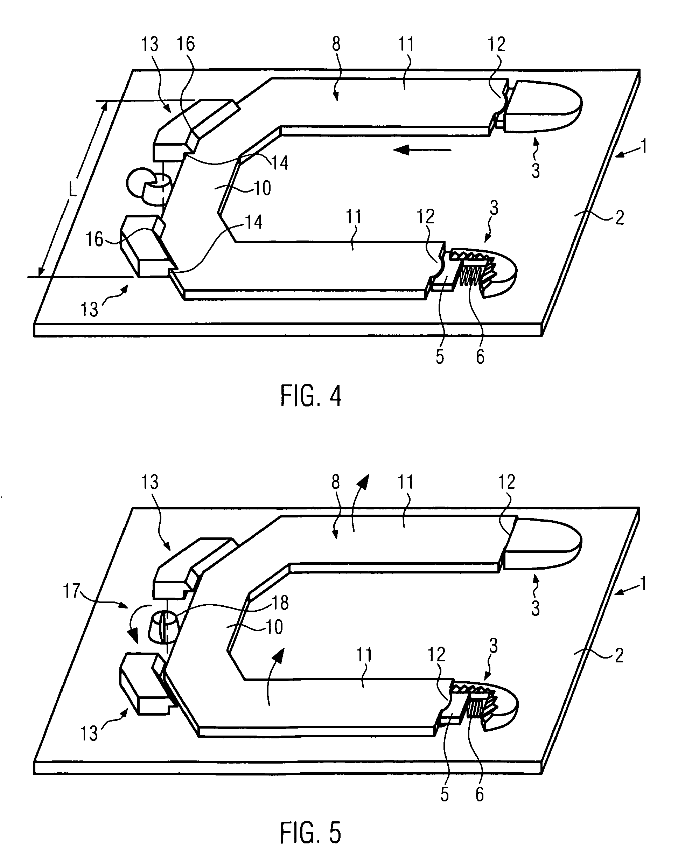

In Figure 4 the third mounting step of the exemplary head immobilization device is shown.

The open frame element 8 is pushed in a direction toward the base fixture elements 13 by the bias exerted by the leg fixture elements 3. The L-shaped profiled edges 14 of the base fixture elements 13 provide a form fit over a distance L with the base portion 10, so that the base portion 10 of the open frame element 8 is prevented from lifting off the base plate structure 1. Furthermore the open frame element 8 is clamped between the profiled edges 14 and the leg fixture elements 3, since the leg fixture elements 3 exert the bias on the end portions 12 of the legs 11 into the direction of the base fixture elements 13. Thus the head fixation element 7 is mounted rigidly to the support plate structure 1.

In Figure 5 the release step of the exemplary head immobilization device is shown.

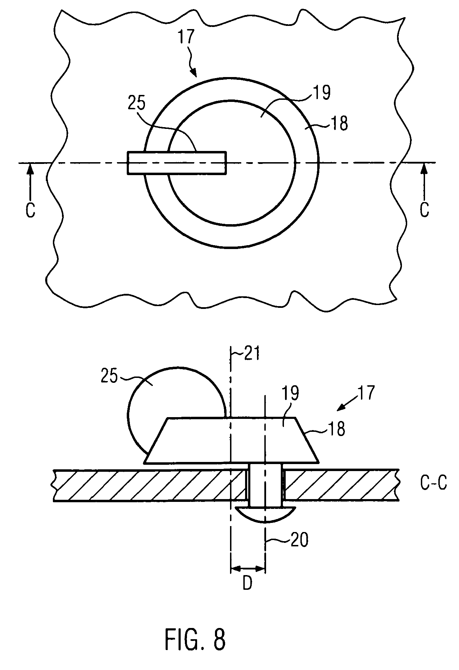

The release element 17 is activated, such that the sloped surface section 18 comes into contact with the base portion 10 of the open frame element 8. The open frame element 8 is pushed against the bias towards the leg fixture elements 3 and a lifting force is introduced by the sloped surface section 18 to the base portion 10. Thereby the open frame element 8 is pushed upward and the head fixation element is released. The release element is described in more detail in Figure 8.

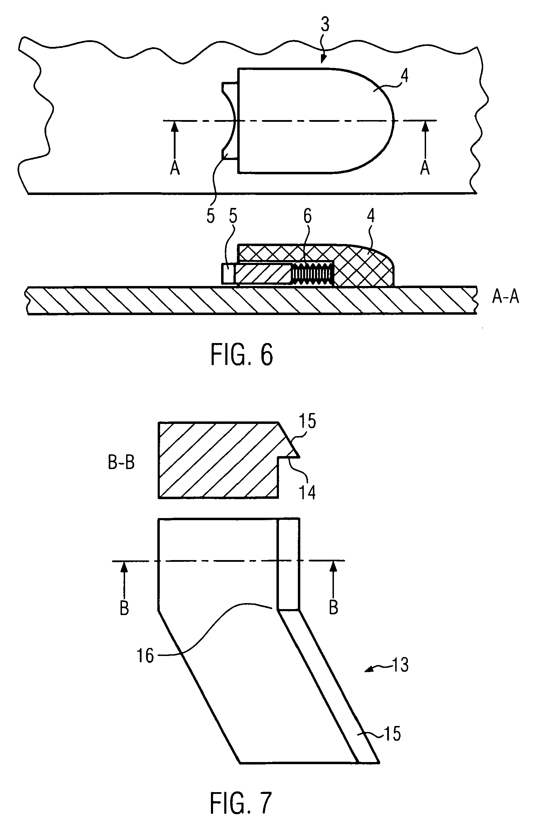

Figure 6 illustrates an embodiment of a leg fixture element 3. A profiled section A-A is shown. The guidance element 4 provides four guiding surfaces in order to guide the receptacle element 5. In this example the elastic element 6 is made from a foamy (elastical) rubber material. The receptacle elements 5 are made from plastic material and provide a concave surface to connect to the end portions 12 (not shown).

Figure 7 shows an embodiment of the base fixture element 13. The L-shaped profile 14 is illustrated as well as the chamfered edge 15 in the profiled section B-B. The complementary contoured rim portion 16 is carried out as a lateral kink in Figure 7.

Figure 8 shows an embodiment of the release element 17 including a profiled section C-C. The release element 17 comprises a sloped surface section 18 with the shape of a truncated cone 19. The pivot axis 20 is parallel to the truncated cone axis 21 at a distance D. When a manual force is exerted on the handle element 25 to rotate the release element 17 about the pivot axis 20, the truncated cone 19 is swiveled towards the base portion 10 of the open frame element 8 (not shown).

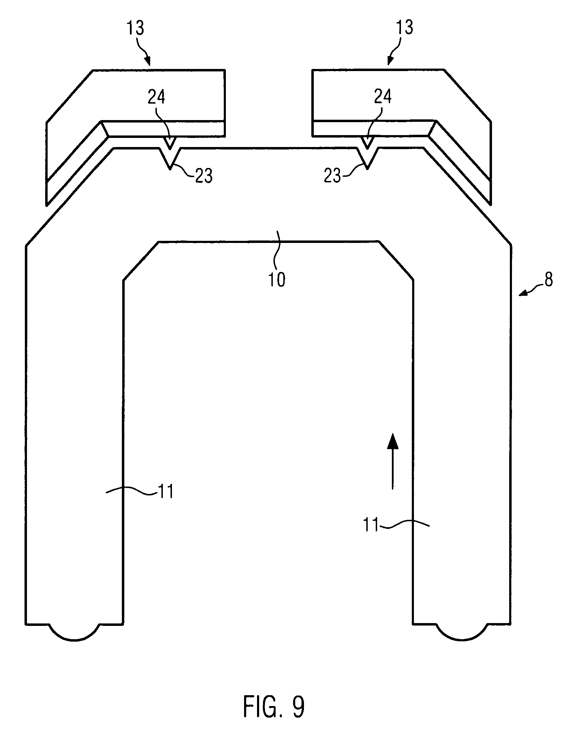

Figure 9 illustrates an embodiment of the guidance notch 23 and the positioning element 24. The base fixture elements 13 comprise a V-shaped positioning element 24. When the open frame element 8 is moved towards the base fixture elements 13 by the bias, the complementary shaped guidance notches 23 are positioned accurately onto the V-shaped positioning elements 24. Thus the open frame element 8 is positioned accurately relative to the base fixture elements 13.

## Claims

1. A head immobilization device for use in the medical field, comprising
a head fixation element (7), comprising a mask element (9) and an open frame element (8), said open frame element (8) comprising a base portion (10) and two legs (11), each having an end portion (12), and
a support plate structure (1), comprising at least one base fixture element (13) for fixing the base portion (10) and at least two leg fixture elements (3) for fixing the legs (11), whereby said head fixation element (7) can be releasably connected with the support plate structure (1),
**characterized in that**
the at least two leg fixture elements (3) are designed to cooperate with the end portions (12) of the legs (11) such as to bias the head fixation element (7) in the direction of the at least one base fixture element (13).

2. A head immobilization device according to claim 1, wherein at least one leg fixture element (3) comprises an elastic element (6).

3. A head immobilization device according to claim 1 and/or 2, wherein the support plate structure (1) comprises a release element (17) designed to exert a counterforce on the head fixation element (7) against the bias.

4. A head immobilization device according to claim 3, wherein the release element (17) comprises a sloped surface section (18), which generates a lifting force on the head fixation element (7), when the release element (17) is activated.

5. A head immobilization device according to claim 4, wherein the sloped surface section (18) comprises the shape of at least one sector of a truncated cone (19).

6. A head immobilization device according to claim 5, wherein the release element (17) comprises a pivot axis (20), whereby the pivot axis (20) comprises an offset (D) to the axis (21) of the truncated cone.

7. A head immobilization device according to any one of the previous claims, wherein the at least one base fixture element (13) comprises at least one profiled edge (14), in particularly wherein the at least one profiled edge (14) comprises an L-shaped and/or C-shaped profile.

8. A head immobilization device according to any one of the previous claims, wherein the at least one base fixture element (13) comprises at least one chamfered edge portion (15).

9. A head immobilization device according to any one of the previous claims, wherein the base portion (10) of the open frame element (8) comprises at least one contoured shoulder portion (22) and wherein the at least one base fixture element (13) comprises at least one complementary contoured rim portion (16).

10. A head immobilization device according to any of the previous claims, wherein the open frame element (8) comprises at least one guidance notch (23) and wherein the support plate structure (1) and/or the base fixture element (13) comprises at least one positioning element (24), wherein the at least one guidance notch (23) interacts with the at least one positioning element (24).

## Patentansprüche

1. Kopf-Immobilisierungsvorrichtung zum Einsatz in der Medizin, die umfasst:
ein Kopf-Fixierelement, das ein Maskenelement (9) sowie ein offenes Rahmenelement (8) umfasst, wobei das offene Rahmenelement (8) einen Basisabschnitt (10) und zwei Schenkel (11) umfasst, die jeweils einen Endabschnitt (12) haben, sowie
eine Trageplattenstruktur (1), die wenigstens ein Basis-Fixierelement (13) zum Fixieren des Basisabschnitts sowie zwei Schenkel-Fixierelemente (3) zum Fixieren der Schenkel (11) umfasst, wobei das Kopf-Fixierelement (7) lösbar mit der Trageplattenstruktur (1) verbunden werden kann,
**dadurch gekennzeichnet, dass**
die wenigstens zwei Schenkel-Fixierelemente (3) so eingerichtet sind, dass sie mit den Endabschnitten (12) der Schenkel (11) so zusammenwirken, dass sie das Kopf-Fixierelement (7) in der Richtung des wenigstens einen Basis-Fixierelementes (13) spannen.

2. Kopf-Immobilisierungsvorrichtung nach Anspruch 1, wobei wenigstens ein Schenkel-Fixierelement (3) ein elastisches Element (6) umfasst.

3. Kopf-Immobilisierungsvorrichtung nach Anspruch 1 und/oder 2, wobei die Trageplattenstruktur (1) ein Löseelement (17) umfasst, das dazu dient, gegen die Spannung eine Gegenkraft auf das Kopf-Fixierelement (7) auszuüben.

4. Kopf-Immobilisierungsvorrichtung nach Anspruch 3, wobei das Löseelement (17) einen abgeschrägten Flächenabschnitt (18) umfasst, der eine Hebekraft an dem KopfFixierelement (7) erzeugt, wenn das Löseelement (17) aktiviert wird.

5. Kopf-Immobilisierungsvorrichtung nach Anspruch 4, wobei der abgeschrägte Flächenabschnitt (18) die Form wenigstens eines Sektors eines Kegelstumpfes (19) umfasst.

6. Kopf-Immobilisierungsvorrichtung nach Anspruch 5, wobei das Löseelement (17) eine Schwenkachse (20) umfasst und die Schwenkachse (20) einen Versatz (D) zu der Achse (21) des Kegelstumpfes umfasst.

7. Kopf-Immobilisierungsvorrichtung nach einem der vorangehenden Ansprüche, wobei das wenigstens eine Basis-Fixierelement (13) wenigstens eine Profilkante (14) umfasst und die wenigstens eine Profilkante (14) ein L-förmiges und/oder C-förmiges Profil umfasst.

8. Kopf-Immobilisierungsvorrichtung nach einem der vorangehenden Ansprüche, wobei das wenigstens eine Basis-Fixierelement (13) wenigstens einen angeschrägten Kantenabschnitt (15) umfasst.

9. Kopf-Immobilisierungsvorrichtung nach einem der vorangehenden Ansprüche, wobei der Basisabschnitt (10) des offenen Rahmenelementes (8) wenigstens einen Kontur-Schulterabschnitt (22) umfasst und das wenigstens eine Basis-Fixierelement (13) wenigstens einen komplementären Kontur-Randabschnitt (16) umfasst.

10. Kopf-Immobilisierungsvorrichtung nach einem der vorangehenden Ansprüche, wobei das offene Rahmenelement (8) wenigstens eine Führungskerbe (23) umfasst und die Trageplattenstruktur (1) und/oder das Basis-Fixierelement (13) wenigstens ein Positionierelement (24) umfassen/umfasst, und wobei die wenigstens eine Führungskerbe (23) mit dem wenigstens einen Positionierelement (24) in Wechselwirkung ist.

## Revendications

1. Dispositif d'immobilisation de tête destiné à être utilisé dans le domaine médical, comprenant
un élément de fixation de tête (7) comprenant un élément de masque (9) et un élément formant cadre ouvert (8), ledit élément formant cadre ouvert (8) comprenant une partie de base (10) et deux pattes (11), possédant chacune une partie d'extrémité (12), et
une structure de plaque support (1) comprenant au moins un élément de fixation de base (13) pour fixer la partie de base (10) et au moins deux éléments de fixation de patte (3) pour fixer les pattes (11), de sorte que ledit élément de fixation de tête (7) peut être relié de manière amovible avec la structure de plaque support (1),
**caractérisé en ce que**
les au moins deux éléments de fixation de patte (3) sont conçus pour coopérer avec les parties d'extrémité (12) des pattes (11) de façon à pousser l'élément de fixation de tête (7) dans la direction de l'au moins un élément de fixation de base (13).

2. Dispositif d'immobilisation de tête selon la revendication 1, dans lequel au moins un élément de fixation de patte (3) comprend un élément élastique (6).

3. Dispositif d'immobilisation de tête selon la revendication 1 et/ou 2, dans lequel la structure de plaque support (1) comprend un élément de libération (17) conçu pour exercer une contre-force sur l'élément de fixation de tête (7) à l'encontre de la poussée.

4. Dispositif d'immobilisation de tête selon la revendication 3, dans lequel l'élément de libération (17) comprend une section de surface inclinée (18), qui génère une force de levage sur l'élément de fixation de tête (7) lorsque l'élément de libération (17) est activé.

5. Dispositif d'immobilisation de tête selon la revendication 4, dans lequel la section de surface inclinée (18) comprend la forme d'au moins un secteur d'un tronc de cône (19).

6. Dispositif d'immobilisation de tête selon la revendication 5, dans lequel l'élément de libération (17) comprend un axe de pivotement (20) tel que l'axe de pivotement (20) comprend un décalage (D) par rapport à l'axe (21) du tronc de cône.

7. Dispositif d'immobilisation de tête selon l'une quelconque des revendications précédentes, dans lequel l'au moins un élément de fixation de base (13) comprend au moins un bord profilé (14), en particulier dans lequel l'au moins un bord profilé (14) comprend un profil en forme de L et/ou en forme de C.

8. Dispositif d'immobilisation de tête selon l'une quelconque des revendications précédentes, dans lequel l'au moins un élément de fixation de base (13) comprend au moins une partie de bord chanfreinée (15).

9. Dispositif d'immobilisation de tête selon l'une quelconque des revendications précédentes, dans lequel la partie de base (10) de l'élément formant cadre ouvert (8) comprend au moins une partie d'épaulement profilée (22) et dans lequel l'au moins un élément de fixation de base (13) comprend au moins une partie de rebord profilée complémentaire (16).

10. Dispositif d'immobilisation de tête selon l'une quelconque des revendications précédentes, dans lequel l'élément formant cadre ouvert (8) comprend au moins une encoche de guidage (23) et dans lequel la structure de plaque support (1) et/ou l'élément de fixation de base (13) comprennent au moins un élément de positionnement (24), dans lequel l'au moins une encoche de guidage (23) interagit avec l'au moins un élément de positionnement (24) .
